# EUROPEAN PATENT SPECIFICATION

(11) **EP 2 764 146 B1**
(45) Date of publication and mention of the grant of the patent: **22.02.2017**
(21) Application number: 12806090.2
(22) Date of filing: 05.12.2012
(51) Int. Cl.: D01F 9/04

(54) **POLYSACCHARIDE FIBRES FOR WOUND DRESSINGS**
POLYSACCHARIDFASERN FÜR WUNDVERBÄNDE
FIBRES POLYSACCHARIDIQUES POUR PANSEMENTS POUR PLAIES

(43) Date of publication of application: 13.08.2014
(73) Proprietor: University of Bolton, Bolton, Lancashire BL3 5AB (GB)
(72) Inventor: MIRAFTAB, Mohsen, Wilmslow Cheshire SK9 4JJ (GB); MASOOD, Rashid, 37610 Faisalabad (PK)
(74) Representative: Wilson Gunn
(86) International application number: PCT/GB2012/053011
(87) International publication number: WO 2013/050794

(56) References cited:
- WO-A1-00/74501
- US-A1- 2006 165 824
- US-A1- 2008 085 354

## Description

The present invention relates to polysaccharide fibres containing polysaccharide polymer derived from Psyllium husk and silver salt. The fibre of the present invention is particularly useful for biomedical applications such as wound management.

Increasing demand for special fibres produced from natural polymers is a function of their unique properties and growing areas of application. Polysaccharides are biologically produced materials that have a unique combination of functional properties and environmentally friendly features. Polysaccharides are polymers with long, chainlike structures. They provide good mechanical properties for applications as fibres, films, adhesives, thickeners, hydrogels, drug delivery agents, emulsifiers, etc. They are natural materials, produced from other biological compounds, and generally are non-toxic and biodegradable. These features make these polysaccharide materials a natural fit for sustainable development. These polysaccharides have also been considered promising materials for healthcare due to their biocompatibility, non-toxicity as well as their ease of use and ability to be easily fabricated into many different forms of products.

Psyllium is a natural polysaccharide obtained from the plantago ovata plant and has been used in many herbal remedies. It is a white fibrous material, partially soluble, hydrophilic in nature and has bulk laxative properties due to its 20 fold swelling capability when brought in contact with water, and forms a gelatinous mass. Its gelatinous mass is soluble in a dilute alkaline solution and re-gels upon acidification.

Psyllium has been widely used in the food and healthcare industry particularly for lowering cholesterol and to promote regular bowel function. FDA has also acknowledged the properties of psyllium as another significant dietary fibre that could affect blood lipids and lower the risk of Coronary Heart Disease.

Alginate is another natural polysaccharide that is produced commercially from seaweed. Over the last two decades, alginate fibres have become well established in the wound management industry where their ion exchange and gel forming abilities are particularly useful in the treatment for exuding wounds. It is biocompatible, biodegradable material and non-toxic to human body. Courtesy of its ionic substituents, alginate possesses very unique and useful properties. In particular, sodium alginate is water soluble while its calcium salt is water insoluble, although highly swelled. Conversion between the two salt forms is reversible by any ion exchange process. Aqueous sodium alginate solutions can be mixed with other materials. This material, either in wet form or after drying is very useful and can be utilized in different biologically controlled processes for specific purpose. Alginate fibres are commonly made by extruding sodium alginate solution into a calcium chloride bath, producing calcium alginate fibres.

The calcium alginate fibre is also porous material and can also be used as drug delivery agent. Calcium alginate can be used to immobilize and protect an active ingredient for storage and release under subsequent conditions. Similarly, calcium alginate can hold a component in an insoluble form while in use. It can be dissolved away when no longer required in the insoluble form. Alginate is an excellent delivery agent with valuable material properties due to its polymeric character, such as mechanical strength and adhesion.

Both alginate and psyllium are hydrophilic in nature and have intrinsic water absorbing properties, especially psyllium which has promising water absorbing properties and absorbs more than 50% of its own weight. There have been a number of reports of psyllium having wound healing properties in addition to water absorption and cholesterol lowering that would make psyllium a potentially viable commercial product. One of the main advantages that psyllium has compared to commercially available water absorbing materials is its low cost and the abundant availability of the raw material. The present invention is based on the realisation that a conjugate fibre of alginate and psyllium that has good textile processability, and is of uniform composition along the length of the produced fibres can be made at a lower cost than other commercially available products.

The property of gel fraction solubility of psyllium in dilute alkaline solution and re-gelling with acid has shown its suitability for use with other fibre forming polysaccharides especially alginate.

According to one aspect of the invention therefore, there is provided a polysaccharide fibre being an elongate fibre having, as the only structural components of the same fibre, alginate and psyllium polymers.

Preferably, the alginate and psyllium polymers are bonded to each other. Thus, the fibre may be produced by extrusion or spinning of a mixture of the alginate and psyllium polymers.

The fibres of the invention can be used in biomedical applications, particularly wound management and may have improved tensile properties and superior liquid absorbency compared to pure alginate fibres.

Preferably the fibre also incorporates an antimicrobial substance or substances, particularly silver substances. Thus, there is provided an antimicrobial fibre containing alginate, psyllium and a silver substance. In this way, a truly antimicrobial conjugate fibre with excellent absorption properties can be made.

The silver substances are preferably incorporated as a water soluble compound i.e. silver nitrate and a partially soluble organic silver compound i.e. silver carbonate.

Most preferably the alginate is incorporated in the form of a water soluble alginate, particularly sodium alginate, and the fibre is formed by extrusion into a coagulating bath which converts the alginate to an insoluble salt, particularly a calcium chloride bath.

In accordance with a further aspect of the invention there is provided a method of making a polysaccharide fibre comprising spinning or extruding an aqueous mixture of soluble psyllium and alginate polymers into a bath containing a substance which coagulates the aqueous mixture to form a fibre.

The procedure of the invention may comprise the steps of extruding a solution of sodium alginate, psyllium and silver compounds (silver nitrate and silver carbonate) directly into a coagulation bath of 1-2% calcium chloride. The conjugate fibre thus formed can then be washed by being passed through a water bath followed by a number of baths containing acetone-water mixtures to bring about water-solvent exchange within the fibres. The purpose of this step is to facilitate drying of the fibres using hot air, after which the fibres are wound up and may be further processed into non-woven felts or ropes or other structures commonly used for wound management.

The first stage of the fibre production process is to have a controlled degree of purification of psyllium gel. It is not feasible to extrude alginate/psyllium fibres in raw form because of psyllium/husk contamination and un-dissolved contents of raw psyllium. Therefore, psyllium gel extraction using cold water, hydrolysis with a base such as sodium hydroxide, and heat may be used in order to fully dissolve the psyllium for extrusion with alginate. The effect of heat is to cause both depolymerisation and solubilisation to a controlled degree.

Although a range of available sodium alginates could be used, the preferred sodium alginate used to produce the conjugate fibres of the invention is Protanal LF 10/60 (supplied by Pronova, Norway) with a high guluronic ratio (ManA 25-35%, GulA 65-75%) or solutions of varying concentration may be used, typically 1 to 6% (w/v), more preferably 2-5% (w/v), most preferably 3-4% (w/v).

The sodium alginate and psyllium polymer solutions are typically ejected under pressure of around 2.2 bar through a spinneret having a number of apertures of defined diameter e.g. 40 to 200 holes with an average diameter of 50µm into a coagulation bath of 1-2% Calcium Chloride. The alginate and psyllium dope is delivered into the 1-2% Calcium Chloride bath at the rate of ∼ 5cm³/min and drawn up to 100% by pick up rollers before being washed in normal water and dried by passing through baths of acetone-water mixtures with increasing concentrations of acetone in each bath i.e. the first bath contains 50% acetone, the second 70% acetone and the final bath 100% acetone. This enables the fibres to be dried using hot air (60-80°C) as they are wound onto a spool.

The properties of fibres produced by this method are found to have improved absorption characteristics when compared to calcium alginate and to be stronger in terms of force to break in comparison with calcium alginate. The tenacity of alginate/psyllium fibres produced according to the method of this invention lies in the range between 2.8 to 11.5 cN/tex, whereas typical values for calcium alginate fibres are between 2-10 cN/tex.

The absorption of alginate/psyllium composite fibres produced according to the method of this invention lies in the range from 16-25 (g/g) for water and 25-35 (g/g) for saline. The fibres diameter also enlarges dramatically upon exposure to either water or saline solutions. Corresponding values for calcium alginate fibres are 6-10 (g/g) water and 9-12 (g/g) saline.

The produced fibres have distinctive morphological characteristics i.e. soft surface with near perfect uniformity resembling surface characteristics of silk fibres. These features are distinctly different to those of pure alginate.

Furthermore, these properties can also be manipulated by including other additives to enhance performance and functional requirements. Examples of additives that may be incorporated into the fibres of the invention either during the production of the fibres or by subsequent post treatment are antimicrobial agents such as silver ions, chlorhexidine or any antibiotic drug, agents known to influence wound healing such as zinc ions, aloe vera or salts of hyaluronic acid and fragrances such as lavender or oil of rosemary.

The invention will now be described by means of the following non-limiting examples.

Example 1: 3-6 g of psyllium was soaked in 1 litre of deionised water for 2 hours. The soaked psyllium was heated up to boiling point for 40-60 minutes. The solution was stirred and filtered in order to separate the un-dissolved particles. 4-4.5% sodium alginate was added and stirring continued for a further 4 hours. The solution was then extruded into 1-2% Calcium Chloride. Extruded fibres were drawn, washed in cold water and passed through acetone water mixtures of increasing acetone concentrations i.e. 50-100% before being dried by hot air. The properties of the fibres when tested were tenacity of 3.3-6.0 cN/tex, elongation at break 5.3-12.4%, water absorption 18-25g/g and saline absorption 20-30g/g.

Example 2: 10-15g of psyllium was soaked in 1 litre of deionised water with 0.1%-0.5% NaOH for 2 hours. The soaked psyllium was heated up at low temperature 50-60°C for 20-30 minutes. The solution was stirred and filtered in order to separate the un-dissolved particles. 4-4.5% sodium alginate was added and stirring continued for a further 4 hours. The solution was then extruded into 1-2% Calcium Chloride. Extruded fibres are drawn, washed in cold water and passed through acetone water mixtures of increasing acetone concentrations i.e. 50-100% before being dried by hot air. Recorded fibre properties were more or less similar to those in example one.

Example 3: 5-15 g of psyllium was soaked in 1 litre of deionised water for 1-2 hours. The soaked psyllium was stirred for 1 hour and filtered in order to separate the un-dissolved particles. 4-4.5% sodium alginate was added and stirring continued for a further 4 hours. The solution was then extruded into 1-2% Calcium Chloride. Extruded fibres are drawn, washed in cold water and passed through acetone water mixtures of increasing acetone concentrations i.e. 50-100% before being dried by hot air. The properties of the fibres when tested were tenacity of 5.5-11.0 cN/tex, elongation at break 7.1-11.4%, water absorption 18-25g/g and saline absorption 20-33g/g.

Example 4: 3-6 g of psyllium was soaked in 1 litre of deionised water for 2 hours. 0.12-0.60 g of silver nitrate was dissolved in 200 ml and boiled for 20-30 minutes separately. The soaked psyllium was added to boiling silver nitrate solution and left on the heat for another 30 minute while stirring. The solution was stirred and filtered in order to separate the un-dissolved particles. 3-4 % sodium alginate was added and stirring continued for a further 4 hours. The solution was then extruded into 1-2% Calcium Chloride. Extruded fibres were drawn, washed in cold water and passed through acetone water mixtures of increasing acetone concentrations i.e. 50-100% before being dried by hot air. Recorded fibre properties are similar to those in Example 1.

Example 5: 5-15 g of psyllium was soaked in 1 litre of deionised water for 1-2 hours. The soaked psyllium was stirred for 1 hour and filtered in order to separate the un-dissolved particles.0.12-1.00 g of silver carbonate was added and stirring continued for another hour. 4.0-4.5% sodium alginate was added and stirring continued for a further 4 hours. The solution was then extruded into 1-2% Calcium Chloride. Extruded fibres are drawn, washed in cold water and passed through acetone water mixtures of increasing acetone concentrations i.e. 50-100% before being dried by hot air. The properties of the fibres when tested were tenacity of 4.19-12.0 cN/tex, elongation at break 7.1-10.4%, water absorption 18-25g/g and saline absorption 20-28g/g.

The invention is not intended to be restricted to the details of the above Examples.

## Claims

1. A polysaccharide fibre being an elongate fibre having, as the only structural components of the same fibre, alginate and psyllium polymers.

2. A fibre according to claim 1 wherein the alginate and psyllium polymers are bonded to each other.

3. A fibre according to claim 1 or 2 which is produced by extrusion or spinning of a mixture of the alginate and psyllium polymers.

4. A fibre according to any one of claims 1 to 3 further incorporating at least one antimicrobial substance.

5. A fibre according to claim 4 wherein the at least one antimicrobial substance comprises at least one silver substance, such as silver nitrate and/or silver carbonate.

6. A method of making a polysaccharide fibre according to any preceding claim, the method comprising spinning or extruding an aqueous mixture of soluble psyllium and alginate polymers into a bath containing a substance which coagulates the aqueous mixture to form a fibre.

7. A method according to claim 6 wherein the coagulating substance comprises a calcium salt which forms insoluble calcium alginate with the soluble alginate polymer of the aqueous mixture.

8. A method according to claim 6 or 7 wherein the bath contains 1-2% by weight calcium chloride.

9. A method according to any one of claims 6 to 8 wherein the soluble psyllium polymer is solubilised in alkaline solution.

10. A method according to any one of claims 6 to 9 wherein the soluble alginate polymer comprises sodium alginate.

11. A method according to any one of claims 6 to 10 wherein the formed fibre is washed with aqueous acetone.

12. A method according to any one of claims 6 to 11 further including at least one antimicrobial substance in the aqueous mixture.

13. A method according to claim 12 wherein the at least one antimicrobial substance comprises at least one silver substance, such as silver nitrate and/or silver carbonate.

14. A method according to any one of claims 6 to 13 wherein the aqueous mixture is formed by first dissolving psyllium polymer and then adding alginate polymer to the psyllium polymer solution.

15. A method according to claim 14 comprising the step of filtering undissolved particles from the psyllium polymer solution prior to said adding of alginate polymer thereto.

## Patentansprüche

1. Polysaccharidfaser, bei der es sich um eine längliche Faser handelt, die als einzige Bausteine derselben Faser Alginat- und Psyllium-Polymere aufweist.

2. Faser nach Anspruch 1, wobei die Alginat- und Psyllium-Polymere aneinander gebunden sind.

3. Faser nach Anspruch 1 oder 2, die durch Extrusion oder Spinnen einer Mischung aus den Alginat- und Psyllium-Polymeren hergestellt wird.

4. Faser nach einem der Ansprüche 1 bis 3, die ferner mindestens eine antimikrobielle Substanz einschließt.

5. Faser nach Anspruch 4, wobei die mindestens eine antimikrobielle Substanz mindestens eine Silbersubstanz wie beispielsweise Silbernitrat und/oder Silbercarbonat umfasst.

6. Verfahren zur Herstellung einer Polysaccharidfaser nach einem vorhergehenden Anspruch, wobei das Verfahren das Spinnen oder Extrudieren einer wässrigen Mischung aus löslichen Psyllium- und Alginat-Polymeren in ein Bad umfasst, das eine Substanz enthält, die die wässrige Mischung koaguliert, um eine Faser zu bilden.

7. Verfahren nach Anspruch 6, wobei die koagulierende Substanz ein Kalziumsalz umfasst, das unlösliches Kalzium-Alginat mit dem löslichen Alginat-Polymer der wässrigen Mischung bildet.

8. Verfahren nach Anspruch 6 oder 7, wobei das Bad 1-2 Gewichts-% Kalziumchlorid enthält.

9. Verfahren nach einem der Ansprüche 6 bis 8, wobei das lösliche Psyllium-Polymer in alkalischer Lösung solubilisiert wird.

10. Verfahren nach einem der Ansprüche 6 bis 9, wobei das lösliche Alginat-Polymer Natrium-Alginat umfasst.

11. Verfahren nach einem der Ansprüche 6 bis 10, wobei die gebildete Faser mit wässrigem Aceton gewaschen wird.

12. Verfahren nach einem der Ansprüche 6 bis 11, ferner mindestens eine antimikrobielle Substanz in der wässrigen Mischung einschließend.

13. Verfahren nach Anspruch 12, wobei die mindestens eine antimikrobielle Substanz mindestens eine Silbersubstanz wie beispielsweise Silbernitrat und/oder Silbercarbonat umfasst.

14. Verfahren nach einem der Ansprüche 6 bis 13, wobei die wässrige Mischung durch zuerst Lösen von Psyllium-Polymer und dann Zugeben von Alginat-Polymer zu der Psyllium-Polymerlösung gebildet wird.

15. Verfahren nach Anspruch 14, den Schritt des Filterns ungelöster Partikel aus der Psyllium-Polymerlösung vor dem Zugeben von Alginat-Polymer zu dieser umfassend.

## Revendications

1. Fibre de polysaccharide, à savoir une fibre allongée possédant, à titre de composants uniques de structure de la même fibre, des polymères d'alginate et de psyllium.

2. Fibre selon la revendication 1, dans laquelle les polymères d'alginate et de psyllium sont liés l'un à l'autre.

3. Fibre selon la revendication 1 ou 2, que l'on obtient par extrusion ou par filage d'un mélange des polymères d'alginate et de psyllium.

4. Fibre selon l'une quelconque des revendications 1 à 3, englobant en outre au moins une substance antimicrobienne.

5. Fibre selon la revendication 4, dans laquelle ladite au moins une substance antimicrobienne comprend au moins une substance à base d'argent telle que le nitrate d'argent et/ou le carbonate d'argent.

6. Procédé de fabrication d'une fibre de polysaccharide selon l'une quelconque des revendications précédentes, le procédé comprenant le filage ou l'extrusion d'un mélange aqueux de polymères solubles de psyllium et d'alginate dans un bain contenant une substance qui coagule le mélange aqueux pour obtenir une fibre.

7. Procédé selon la revendication 6, dans lequel la substance de coagulation comprend un sel de calcium qui forme de l'alginate de calcium insoluble avec le polymère d'alginate soluble du mélange aqueux.

8. Procédé selon la revendication 6 ou 7, dans lequel le bain contient de 1 à 2 % en poids de chlorure de calcium.

9. Procédé selon l'une quelconque des revendications 6 à 8, dans lequel le polymère de psyllium soluble est solubilisé dans une solution alcaline.

10. Procédé selon l'une quelconque des revendications 6 à 9, dans lequel le polymère d'alginate soluble comprend de l'alginate de sodium.

11. Procédé selon l'une quelconque des revendications 6 à 10, dans lequel on lave la fibre obtenue avec de l'acétone aqueux.

12. Procédé selon l'une quelconque des revendications 6 à 11, englobant en outre au moins une substance antimicrobienne dans le mélange aqueux.

13. Procédé selon la revendication 12, dans lequel ladite au moins une substance antimicrobienne comprend au moins une substance à base d'argent telle que le nitrate d'argent et/ou le carbonate d'argent.

14. Procédé selon l'une quelconque des revendications 6 à 13, dans lequel on obtient le mélange aqueux en dissolvant d'abord le polymère de psyllium et en ajoutant ensuite le polymère d'alginate à la solution du polymère de psyllium.

15. Procédé selon la revendication 14, comprenant l'étape dans laquelle on élimine par filtration des particules non dissoutes de la solution de polymère de psyllium avant d'y ajouter le polymère d'alginate.
